# EUROPÄISCHE PATENTSCHRIFT

(11) **EP 1 290 436 B1**
(45) Veröffentlichungstag und Bekanntmachung des Hinweises auf die Patenterteilung: **17.08.2005**
(21) Anmeldenummer: 01933997.7
(22) Anmeldetag: 21.05.2001
(51) Int. Cl.: G01N 33/15, G01N 3/40, G01G 17/00

(54) **VORRICHTUNG UND VERFAHREN ZUR AUTOMATISCHEN QUALITÄTSKONTROLLE VON PRÜFLINGEN**
DEVICE AND METHOD FOR AUTOMATICALLY CARRYING OUT QUALITY CONTROL OF TEST ITEMS
DISPOSITIF ET PROCEDE SERVANT AU CONTROLE DE QUALITE AUTOMATIQUE D'ECHANTILLONS

(30) Priorität: 21.05.2000 DE 10024598
(43) Veröffentlichungstag der Anmeldung: 12.03.2003
(73) Patentinhaber: Kraemer Elektronik GMBH, 64291 Darmstadt (DE)
(72) Erfinder: KRAEMER, Norbert, 64291 Darmstadt (DE)
(74) Vertreter: Mierswa, Klaus, Dipl.-Ing.
(86) Internationale Anmeldenummer: PCT/EP2001/005827
(87) Internationale Veröffentlichungsnummer: WO 2001/090744

(56) Entgegenhaltungen:
- WO-A-87/00621
- WO-A-98/19945
- DE-A- 3 130 512
- DE-A- 4 241 985
- DE-A- 19 733 436
- US-A- 4 393 717

## Beschreibung

### Technisches Gebiet:

Die Erfindung betrifft eine Vorrichtung zur automatischen Qualitätskontrolle von Prüflingen, wie Tablets, Tabletten, Pillen oder Dragees, zur Bestimmung von mechanisch-physikalischen Eigenschaften derselben, wie Gewicht, Abmessungen, Zerfallszeit in einem Medium sowie Härte, bestehend aus einer Zuführeinrichtung und einem waagrecht angeordneten Transportstern mit peripher angeordneten Aufnahmekammern für je einen Prüfling sowie einem mittels eines Elektromotors angetriebenen Härtetester, gegebenenfalls einer Waage sowie einer Einrichtung zur Bestimmung der Abmessungen des Prüflings, wobei an einen Abgang des Transportsterns zur Übernahme des Prüflings tangential ein Linearförderer angeschlossen ist, an den sich der Härtetester anschließt, gemäß Patentanspruch 1. Ebenso betrifft die Erfindung ein Verfahren gemäß Patentanspruch 7.

### Stand der Technik:

Im Rahmen der Qualitätskontrolle bei der Fertigung von Tabletten, Pillen oder Dragees, aber auch bei der Herstellung von Tablets in der Waschmittelindustrie, werden mechanisch-physikalische Eigenschaften derselben, wie Gewicht, Abmessungen, Zerfallszeit in einem Medium und Härte bestimmt. Dazu sind Tablettenprüfsysteme bekannt, welche jeweils eines Mehrzahl von Tabletten aus einem Produktionszyklus auf diese Eigenschaften hin untersuchen. Tabletten einer Charge werden aus einem Vorratsbehälter vereinzelt und z.B. mit einem Transportstern oder Transportband von einer Meßstation zur nächsten befördert. Derartige Transportsteme besitzen peripher in der Regel 24 Kammern zur vereinzelten Aufnahme je eines Tablettenprüflings. Zuerst erfolgt ein Wiegevorgang, an den sich eine Messeinrichtung zur Ermittlungen der Abmessungen des Prüflings anschließt, auf die ein Härtetester folgt. Das Zusammenspiel der einzelnen Stationen sowie die Speicherung und Übermittlung der Messergebnisse übernimmt eine Software sowie eine zentrale Recheneinheit.

Die Härte eines Prüfkörpers wird üblicherweise in einer Kraftmeßdose gemessen, die einen Druckkolben und ein Gegenlager aufweist. Der Prüfkörper, also die Tablette, wird in den Bereich zwischen Druckkolben und Gegenlager befördert, wobei die Tablette vorzugsweise das Gegenlager berührt. Der Druckkolben wird nun mittels eines Schrittmotors gegen das Gegenlager und die vor diesem liegende Tablette gefahren. Die vom Druckkolben mit jedem Schritt des Motors ausgeübte Kraft wird gemessen und aufgezeichnet, die konstant und sehr klein ist, solange der Druckkolben die Tablette nicht berührt oder diese ohne den Gegendruck des Gegenlagers über den Prüftisch schiebt. Wenn der Druckkolben die Tablette berührt und gegen das Gegenlager drückt, steigt die von ihm ausgeübte Kraft mit jedem Schritt des Schrittmotors so lange an, bis die Tablette zerbricht. Die dazu aufgewendete Kraft wird aufgezeichnet und dient als Maß für die Härte der Tablette. Das plötzliche Abfallen der vom Druckkolben aufgewendeten Kraft beim Zerbrechen des Prüfkörpers dient als Abbruchbedingung für das Beenden der Messung. Der Druckkolben wird in seine Ausgangsposition zurückgefahren, und die nächste Tablette kann geprüft werden.

Eine Vorrichtung zur Durchführung eines Härtetests ist durch die WO 98/ 53298 bekannt geworden, die einen Prüftisch zur Aufnahme des Prüfkörpers sowie einen linear verfahrbaren Druckkolben und ein Gegenlager aufweist, die oberhalb des Prüftisches angeordnet und gegeneinander verfahrbar sind. Der Prüfkörper befindet sich zwischen Druckkolben und Gegenlager, wobei die beim Gegeneinanderdrücken von Druckkolben und Gegenlager aufgewendete Kraft oder eine dazu proportionale Größe mittels einer Kraftmeßvorrichtung meßbar und die Härte des Prüfkörpers daraus bestimmt wird.

Ebenso sind Linearförderer für Tabletten oder Pillen in der Form von vor und zurück laufenden sowie heb- und senkbaren Rechen oder Schwingförderern bekannt. So ist durch die WO 85/03278 (US 4,660,713) eine Transporteinrichtung für Tabletten bekannt geworden, bei von einer Zuführeinrichtung zugeführte Tabletten mittels eines vor und zurück laufenden sowie heb- und senkbaren Rechens, der eine V-förmige Gabel aufweist, auf einer horizontalen Führungsbahn zu einer Tablettenprüfstation, einer Verpackungseinrichtung sowie u. U. weiterer Stationen transportiert werden. Die Führungsbahn weist beidseitig des von der Spitze der V-förmigen Gabel überstrichenen Bereichs Öffnungen auf, deren minimaler Abstand so bemessen ist, dass Tabletten mit dem maximalen Durchmesser, der geprüft werden soll, sicher zwischen den Öffnungen hindurch transportiert werden, wenn sie an der Spitze der V-förmigen Gabel anliegen, wobei diejenigen Tabletten, welche beim Transport nicht an der Spitze der V-förmigen Gabel anliegen, in die Öffnungen fallen. Die Öffnungen, die sich in Transportrichtung der Tabletten zur Führungsbahn hin vergrößern, sind in der Führungsbahn über eine Rutsche mit einem Sammelbehälter verbunden.

Durch die DE 199 17 925 A1 ist ein Linearförderer in Form eines Vibrationsförderers bekannt geworden, bei dem zur Befestigung der Schwingplatte in derselben quer zur Förderrichtung Hintergreifnuten angeordnet sind.

Bei der Verwendung von Härtetestern im Rahmen von Vorrichtungen auf der Basis von Transportsternen bestimmt die Station des Härtetesters die Verarbeitungsgeschwindigkeit, welche dadurch limitiert ist. Denn wenn ein Härtetest durchzuführen ist, so muss aufgrund der übrigen in Reihe geschalteten Verarbeitungseinrichtungen die Beendigung des ablaufenden Härtetests abgewartet werden, bis mit einem weiteren Prüfling eine weitere Untersuchung durchgeführt werden kann.

Durch die DE 197 33 436 A1 ist eine Einrichtung zum Positionieren von Tabletten oder ähnlich geformten Prüflingen in den Teststationen eines Tablettenprüfgerätes bekannt geworden, welches einen Karusselverteiler und eine davon radial abgehenden und schräg geneigte Rutsche aufweist, an welche sich unter 90 Grad hierzu ein Rechen zur Bewegung der Tabletten anschließt und diese in eine Teststation befördert, welche auch eine Bruchhärteeinrichtung sein kann. Durch die DE 31 30 512 C2 ist eine ähnlich gestaltete Vorrichtung mit vertikal angeordneter Dreh- und Verteilertrommel bekannt geworden zur Prüfung von Eigenschaften pharmazeutischer Prüflinge.

Durch die US 4,393,717 A ist eine Vorrichtung für die Durchführung eines Härtetests an Tabletten bekannt geworden, welche einen waagrechten Transportstern mit Aufnahmemulden zur Aufnahme je einer Tablette sowie einer tangential angeordneten Ausschleusungsbahn unterhalb des Transportsterns aufweist, welche die einzelnen Tabletten in eine Prüfstation befördert. In der Prüfstation wird die einzelne Tablette, beispielsweise betreffend des Durchmessers, des Gewichts und der Härte geprüft.

### Technische Aufgabe:

Der Erfindung liegt die Aufgabe zugrunde, eine Vorrichtung und ein Verfahren der eingangs genannten Gattungen zu schaffen, mit denen die Durchlaufzeit eines Prüflings vom Eintritt in den Transportstern bis zur Beendigung des Härtetests verkürzt und die Bauart verkleinert werden kann.

### Offenbarung der Erfindung sowie deren Vorteile:

Die Lösung der Aufgabe bei einer Vorrichtung der genannten Gattung besteht darin, dass die Vorrichtung eine Datenverarbeitungseinrichtung zur Aufnahme und Verarbeitung sämtlicher Daten mit einem Hauptprozessor aufweist und dem elektrischen Antriebsmotor des Härtetesters wenigstens ein eigener Zusatzprozessor zugeordnet ist, der mit dem Hauptprozessor zur Koordination der Bewegung des Härtetesters mit dem laufenden Transportstern zusammenarbeitet.

In weiterer Ausgestaltung der Erfindung können auch alle weiteren Einrichtungen, wie Transportstern, Einrichtung zur Bestimmung der Abmessungen des Prüflings sowie Härtetester, welche einen eigenen elektrischen Antriebsmotor aufweisen, wenigstens einen eigenen Zusatzprozessor besitzen zur Vorverarbeitung der in dieser Einrichtung anfallenden Daten und zur Koordination der Bewegungen der entsprechenden Einrichtung mit dem laufenden Transportstern. Auf diese Weise können die einzelnen Prozeßschritte optimal aufeinander abgestimmt werden, woraus eine erhebliche Zunahme der Prozeßgeschwindigkeit ergibt.

Die Vorrichtung besitzt den Vorteil, dass nunmehr die Prozeßzeit zur vollständigen Überprüfung eines Prüflings vom Eintritt in den Transportstern bis zur Beendigung des Härtetests erheblich verkürzt ist. Der Grund liegt darin, weil die normalerweise nur streng nacheinander in Reihe ablaufenden Prozesse oder Prüfschritte, denen eine Prüfling zu unterwerfen ist, nunmehr teilweise parallel ablaufen, indem der Prozeßschritt der Härtemessung von den Prozeßschritten beispielsweise des Verwiegens und des Vermessens von Abmessungen, wie Höhenmessung, des Prüflings getrennt wird. Dadurch können während des Prozeßschrittes der Härtemessung an einem Prüfling weitere nachkommende Prüflinge den vorgeschalteten Prozeßschritten des Verwiegens und der Vermessung von Abmessungen unterworfen werden.

Mit der Abkoppelung der Härtemessung vom Transportstern ist des Weiteren eine erhebliche Verkleinerung des Transportsternes verbunden, so dass die Vorrichtung eine insgesamt kleinere Bauweise wie vergleichbare Geräte des Standes der Technik aufweist. Der Transportstern der Erfindung braucht nur eine erheblich geringere Zahl von Aussparungen zur Aufnahme von Prüflingen aufweisen, so dass der Durchmesser des Transportsterns geringer ist als bei vergleichbaren Vorrichtungen des Standes der Technik. Das ist insbesondere der Fall, wenn der Linearförderer tangential an dem Transportstern angeordnet ist oder sich sogar unterhalb des Transportsterns befindet.

In einer Ausgestaltung der Erfindung ist vor dem Abgang des Transportsterns zum Linearförderer ein weiterer Abgang für diejenigen vor dem Härtetester auszuschleusenden Prüflinge angeordnet. Dieser Abgang für diejenigen Prüflinge, die dem Härtetest nicht unterworfen werden sollen, kann aber auch erst nach dem Abgang zum Härtetester bzw. zum Linearförderer angeordnet sein. Linearförderer und Härtetester bilden bevorzugt eine bauliche Einheit.

Der Linearförderer, der in bekannter Weise einen in Richtung zum Härtetester vor und zurück laufenden sowie heb- und senkbaren Rechen aufweist, besteht aus zwei senkrecht sich gegenüberstehenden Wänden, zwischen denen die Führungsbahn und der Rechen angeordnet sind.

Ein weiterer Vorteil der Trennung des Prozeßschrittes der Härtemessung von den übrigen Prozeßschritten liegt darin, dass kein Staub und keine feinen Bruchstücke der zerborstenen Prüflinge mehr im Transportstern existieren und der Transportstern staubfrei arbeitet.

Das erfindungsgemäße Verfahren zur automatischen Qualitätskontrolle von Prüflingen, wie Tablets, Tabletten, Pillen oder Dragees, zur Bestimmung von mechanisch-physikalischen Eigenschaften derselben, wie Gewicht, Abmessungen, Zerfallszeit in einem Medium sowie Härte, in unterschiedlichen Prozeßschritten, unter Zuhilfenahme einer Zuführeinrichtung und eines Transportsterns mit peripher angeordneten Aufnahmekammern für je einen Prüfling sowie eines Härtetesters, gegebenenfalls einer Waage sowie einer Einrichtung zur Bestimmung der Abmessungen des Prüflings, besteht darin, dass der Prozeßschritt der Härtemessung eines Prüflings von den übrigen Prozeßschritten abgekoppelt wird und dieser Prozeßschritt der Härtemessung eines Prüflings parallel während der übrigen Prozeßschritte an einem weiteren Prüfling ausgeführt wird.

Vorzugsweise ist es möglich, bei der Zuführung eines Prüflings in den Prozeßschritt der Härtemessung eine Mehrzahl weiterer Prüflinge innerhalb des Transportsterns den Prozeßschritten Verwiegen und Vermessung von Abmessungen während der Durchführung des Prozeßschrittes der Härtemessung zu unterwerfen. Aufgrund dieses Arbeitsverfahrens kann die gesamte Verarbeitungsgeschwindigkeit der Vorrichtung erheblich gesteigert werden.

Als Prüflinge kommen nicht nur Tabletten, Pillen oder Dragees der pharmazeutischen Industrie in Frage, sondern gleichermaßen Tablets der Waschmittel- und Reinigungsindustrie. Da derartige Tablets häufig erheblich größere Durchmesser als pharmazeutische Tabletten oder Pillen aufweisen, müssen die Dimensionen des Transportsterns und gegebenenfalls auch des Härtetesters, wie auch weiterer Einrichtungen, entsprechend angepasst sein.

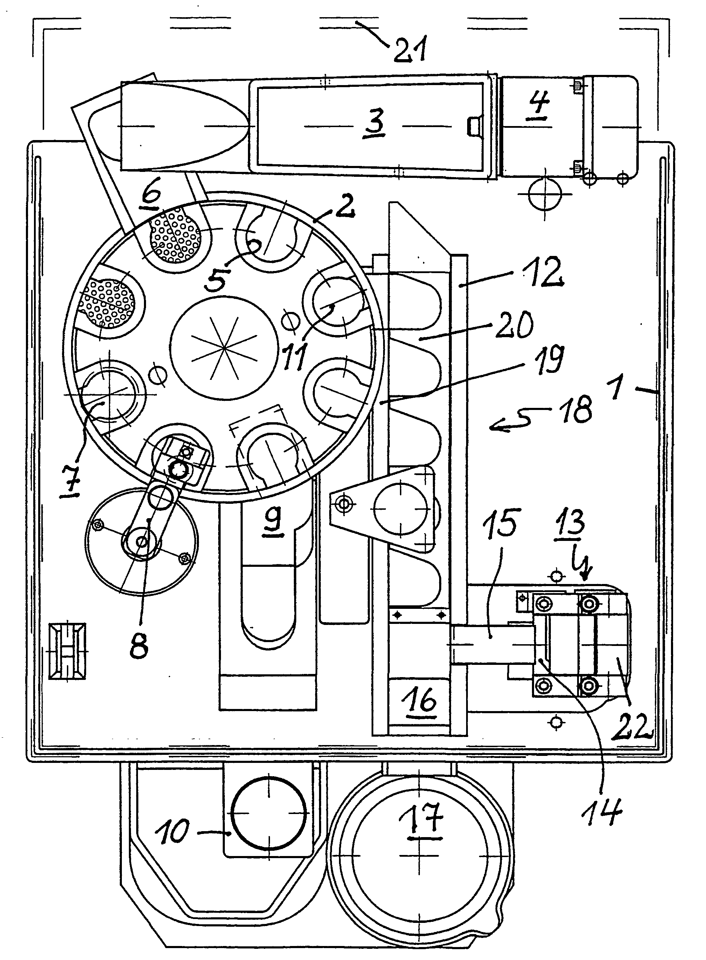

Die Figur zeigt eine schematische Draufsicht auf eine erfindungsgemäße Vorrichtung, bestehend aus einem Gehäuse 1, in dem ein Transportstern 2 drehbar angeordnet ist. Über eine von einem Schrittmotor 4 angetriebene bewegliche Rinne 3 zum Vereinzeln der Prüflinge gelangen dieselben über eine Rutsche 6 vereinzelt in peripher angeordnete Aussparungen 5 des Transportsterns 2. Der rutsche 6 benachbart ist als erste Prozeßstation eine Waage 7, auf welcher der Prüfling gewogen wird. Daran schließt sich eine Einrichtung 8 zu Höhenvermessung des Prüflings an. Nach der Höhenmeßeinrichtung 8 folgt ein erster Abgang 9 aus dem Transportstern, welcher verschließbar und öffenbar ist, über den diejenigen Prüflinge in einen Behälter 10 gelangen, welche nicht dem Prozeßschritt der Härtemessung unterzogen werden sollen.

Nach diesem Abgang 9 folgt ein weiterer Abgang 11, welcher hin zu einem Linearförderer 18 führt. Der Linearförderer 18 ist vorzugsweise tangential am bzw. neben dem Transportstern 2 angeordnet, wie es in der Figur gezeigt ist. Allerdings kann der Linearförderer auch unterhalb des Transportsterns angeordnet sein, was die Bauweise noch zu verkleinern imstande ist.

Der Linearförderer 18, der bevorzugt aus Edelstahl hergestellt ist, besteht aus zwei sich senkrecht gegenüberstehenden Wänden, zwischen denen eine Förderbahn und ein Rechen 20 angeordnet ist. Eine nicht gezeigte Bewegungseinrichtung für den Rechen 20 transportiert denselben, vorzugsweise mittels eines Exzenters, um eine vorgegebene kurze Strecke auf der Förderbahn in Richtung eines Härtetesters 13, der sich an das Ende der Förderbahn bzw. des Linearförderers 18 anschließt. Anschließend wird der Rechen 20 angehoben und im angehobenen Zustand entgegen der ersten Transportrichtung um dieselbe Strecke zurückgeführt und abgesenkt, um erneut um die Strecke auf der Förderbahn verschoben zu werden. Dadurch wird der einzelne Prüfling, der über den Abgang 11 auf die Förderbahn des Linearförderers gelangt ist, vorzugsweise mit einer Mehrzahl von Linearbewegungen auf der Förderbahn in den Härtetester 13 und dort in den Bereich eines beweglich angeordneten Schubbackens 15 bewegt. Der Schubbacken ist an einer Kraftmeßdose 14 befestigt. Über eine motorangetriebene Walze 22 kann der Schubbacken auf die gegenüber liegende Wandung 19 des Linearförderers 18 bewegt werden; Linearförderer 18 und Härtetester 13 bilden bevorzugt eine bauliche Einheit.

Nach der Bestimmung des Durchmessers der Prüflings und nach dem Härtetest werden die verbliebenen Bruchstücke des Prüflings mittels einer weiteren Vorschubbewegung des Rechens 20 auf der Förderbahn über einen Ausgang 16 in einen Behälter 17 entsorgt. Mittels eines durchsichtigen Klappdeckels 21 ist das Gehäuse 1 der Vorrichtung verschließbar.

### Gewerbliche Anwendbarkeit:

Die erfindungsgemäße Vorrichtung und das Verfahren sind insbesondere in der pharmazeutischen und der Waschmittel- und Reinigungsindustrie zur automatischen Qualitätskontrolle von Prüflingen zur Bestimmung von mechanisch-physikalischen Eigenschaften derselben gewerblich anwendbar.

### Liste der Bezugszeichen:

- 1: Gehäuse
- 2: Transportstern
- 3: Zuführeinrichtung, nämlich Rinne
- 4: Schrittmotor
- 5: Aussparungen des Transportsterns
- 6: Rutsche
- 7: Waage
- 8: Höhenmesseinrichtung
- 9, 11: Abgänge
- 10: Behälter
- 12, 19: Wandungen
- 13: Härtetester
- 14: Kraftmeßdose
- 15: Schubbacken
- 16: Ausgang
- 17: Behälter
- 18: Linearförderer
- 20: Rechen
- 21: Deckel
- 22: Walze

## Patentansprüche

1. Vorrichtung zur automatischen Qualitätskontrolle von Prüflingen, wie Tablets, Tabletten, Pillen oder Dragees, zur Bestimmung von mechanisch-physikalischen Eigenschaften derselben, wie Gewicht, Abmessungen, Zerfallszeit in einem Medium sowie Härte, bestehend aus einer Zuführeinrichtung (3,6) und einem waagrecht angeordneten Transportstern (2) mit peripher angeordneten Aufnahmekammern (5) für je einen Prüfling sowie einem mittels eines Elektromotors angetriebenen Härtetester (13), gegebenenfalls einer Waage (7) sowie einer Einrichtung (8) zur Bestimmung der Abmessungen des Prüflings, wobei an einen Abgang (11) des Transportsterns (2) zur Übernahme des Prüflings tangential ein Linearförderer (18) angeschlossen ist, an den sich der Härtetester (13) anschließt,
**dadurch gekennzeichnet, dass** die Vorrichtung eine Datenverarbeitungseinrichtung zur Aufnahme und Verarbeitung sämtlicher Daten mit einem Hauptprozessor aufweist und dem elektrischen Antriebsmotor des Härtetesters (13) wenigstens ein eigener Zusatzprozessor zugeordnet ist, der mit dem Hauptprozessor zur Koordination der Bewegung des Härtetesters (13) mit dem laufenden Transportstern (2) zusammenarbeitet.

2. Vorrichtung nach Anspruch 1, **dadurch gekennzeichnet,**
**dass** die Einrichtungen, wie Transportstern (2), Einrichtung (8) zur Bestimmung der Abmessungen des Prüflings sowie Härtetester (13), die einen eigenen elektrischen Antriebsmotor aufweisen, wenigstens einen eigenen Zusatzprozessor besitzen zur Vorverarbeitung der in dieser Einrichtung anfallenden Daten und Koordination der Bewegungen der entsprechenden Einrichtung mit dem laufenden Transportstern (2).

3. Vorrichtung nach Anspruch 1, **dadurch gekennzeichnet,**
**dass** Linearförderer (18) und Härtetester (13) eine bauliche Einheit bilden.

4. Vorrichtung nach Anspruch 1, **dadurch gekennzeichnet, dass** vor dem Abgang (11) des Transportsterns (2) zum Linearförderer (18) ein weiterer Abgang (9) für diejenigen vor dem Härtetester (13) auszuschleusenden Prüflinge angeordnet ist.

5. Vorrichtung nach Anspruch 1, wobei der Linearförderer (18) einen in Richtung zum Härtetester (13) vor und zurück laufenden sowie heb- und senkbaren Rechen (20) aufweist, **dadurch gekennzeichnet,**
**dass** der Linearförderer aus zwei senkrecht sich gegenüberstehenden Wänden (12,19) besteht, zwischen denen die Führungsbahn und der Rechen (20) angeordnet sind.

6. Vorrichtung nach Anspruch 1, **dadurch gekennzeichnet,**
**dass** der Linearförderer (18) unterhalb des Transportsterns (2) angeordnet ist.

7. Verfahren zur automatischen Qualitätskontrolle von Prüflingen, wie Tablets, Tabletten, Pillen oder Dragees, zur Bestimmung von mechanisch-physikalischen Eigenschaften derselben, wie Gewicht, Abmessungen, Zerfallszeit in einem Medium sowie Härte, in unterschiedlichen Prozeßschritten, unter Zuhilfenahme einer Zuführeinrichtung (3,6) und eines Transportsterns (2) mit peripher angeordneten Aufnahmekammern (5) für je einen Prüfling sowie eines Härtetesters (13), gegebenenfalls einer Waage (7) sowie einer Einrichtung (8) zur Bestimmung der Abmessungen des Prüflings,
**dadurch gekennzeichnet, dass** der Prozeßschritt der Härtemessung eines Prüflings von den übrigen Prozeßschritten abgekoppelt wird und dieser Prozeßschritt der Härtemessung eines Prüflings parallel während der übrigen Prozeßschritte an einem weiteren Prüfling ausgeführt wird.

8. Verfahren nach Anspruch 7, **dadurch gekennzeichnet,**
**dass** bei der Zuführung eines Prüflings in den Prozeßschritt der Härtemessung eine Mehrzahl weiterer Prüflinge innerhalb des Transportsterns den Prozeßschritten Verwiegen und Vermessung von Abmessungen während der Durchführung des Prozeßschrittes der Härtemessung unterworfen werden.

## Claims

1. A device for the automatic quality control of test specimens such as tablets, tabs, pills or dragees, and for determining their mechanical-physical properties such as the weight, the dimensions, the disintegration time in a medium as well as the hardness, said device consisting of a feed means (3, 6) and a horizontally arranged star feeder (2) with peripherally arranged receiving chambers (5), each for one test specimen, as well as a hardness tester (13) driven by an electric motor, optionally a set of scales (7) as well as a means (8) for determining the dimensions of the test specimen, whereby at one outlet (11) of the star feeder (2), there is a line conveyor (18) that is connected tangentially in order to receive the test specimen, upstream from which is the hardness tester (13),
**characterized in that** the device comprises a data processing device for receiving and processing all of the data with a main processor, and the electric drive motor of the hardness tester (13) is associated with at least one additional processor of its own that cooperates with the main processor in order to coordinate the movement of the hardness tester (13) with the running star feeder (2).

2. The device according to Claim 1, **characterized in that**
the means, such as, for example, the star feeder (2), the means (8) for determining the dimensions of the test specimen and the hardness testers (13), which have their own electric drive motor, have at least one additional processor of their own for the preliminary processing of the data acquired in this means and for the coordination of the movements of the means in question with the running star feeder (2).

3. The device according to Claim 1, **characterized in that**
the linear conveyor (18) and the hardness tester (13) form a structural unit.

4. The device according to Claim 1, **characterized in that**,
before the outlet (11) of the star feeder (2) to the linear conveyor (18), there is another outlet (9) for those test specimens that are to be ejected upstream from the hardness tester (13).

5. The device according to Claim 1, whereby the linear conveyor (18) has a rake (20) that can move back and forth as well as up and down in the direction of the hardness tester (13), **characterized in that**
the linear conveyor consists of two perpendicular walls (12, 19) across from each other, between which the guide path and the rake (20) are arranged.

6. The device according to Claim 1, **characterized in that**
the linear conveyor (18) is arranged underneath the star feeder (2).

7. A method for the automatic quality control of test specimens such as tablets, tabs, pills or dragees, and for determining their mechanical-physical properties such as the weight, the dimensions, the disintegration time in a medium as well as the hardness, in different process steps, making use of a feed means (3, 6) and a star feeder (2) with peripherally arranged receiving chambers (5), each for one test specimen, as well as a hardness tester (13), optionally with scales (7), as well as a means (8) for determining the dimensions of the test specimen,
**characterized in that** the process step involving the hardness measurement of a test specimen is uncoupled from the other process steps and this process step of hardness measurement of a test specimen is carried out in parallel during the other process steps using another test specimen.

8. The method according to Claim 7, **characterized in that**,
when a test specimen enters the process step of the hardness measurement, several other test specimens inside the star feeder undergo the process steps involving weighing and measurement of the dimensions while the process step of the hardness measurement is being carried out.

## Revendications

1. Dispositif servant au contrôle de qualité automatique d'échantillons tels que des tablettes, des comprimés, des pilules ou des dragées, à la détermination des caractéristiques physico-mécaniques de ceux-ci, telles que le poids, les dimensions, le temps de désagrégation dans un médium, ainsi que la dureté, consistant en une unité d'alimentation (3, 6) et en une unité de transport en étoile (2) disposée horizontalement avec des chambres (5) disposées en périphérie pour loger chacune un échantillon, ainsi qu'en un testeur de dureté (13) entraîné au moyen d'un moteur électrique, le cas échéant en une balance (7), ainsi qu'en une unité (8) pour déterminer les dimensions de l'échantillon, un convoyeur linéaire (18) étant tangentiellement contigu à l'une des sorties (11) du dispositif de transport en étoile (2) pour la prise en charge de l'échantillon, convoyeur auquel est adjoint le testeur de dureté (13),
**caractérisé en ce que** le dispositif présente un système de traitement des données pour l'enregistrement et le traitement de toutes les données avec un processeur principal et qu'au moins un propre processeur additionnel est assigné au moteur d'entraînement électrique du testeur de dureté (13), lequel processeur additionnel travaille en interaction avec le processeur principal pour coordonner le mouvement du testeur de dureté (13) avec l'unité de transport en étoile (2) en marche.

2. Dispositif selon la revendication 1, **caractérisé en ce que**
les unités telles que l'unité de transport en étoile (2), l'unité (8) pour la détermination des dimensions de l'échantillon, ainsi que le testeur de dureté (13) qui présentent un propre moteur d'entraînement électrique, possèdent au moins un propre processeur additionnel pour le prétraitement des données produites dans cette unité et la coordination des mouvements des unités respectives avec l'unité de transport en étoile (2) en marche.

3. Dispositif selon la revendication 1, **caractérisé en ce que**
le convoyeur linéaire (18) et le testeur de dureté (13) constituent une unité constructive.

4. Dispositif selon la revendication 1, **caractérisé en ce que** avant la sortie (11) de l'unité de transport en étoile (2) sur le convoyeur linéaire (18) une autre sortie (9) est disposée pour les échantillons qui doivent être éliminés avant le testeur de dureté (13).

5. Dispositif selon la revendication 1, le convoyeur linéaire (18) présentant un râteau (20) qui marche en avant et en arrière en direction du testeur de dureté (13), et qui également s'élève et s'abaisse, **caractérisé en ce que**
le convoyeur linéaire consiste en deux parois verticales qui se font face (12, 19), entre lesquelles sont disposés la glissière de guidage et le râteau (20).

6. Dispositif selon la revendication 1, **caractérisé en ce que**
le convoyeur linéaire (18) est disposé au-dessous de l'unité de transport en étoile (2).

7. Procédé servant au contrôle de qualité automatique d'échantillons tels que des tablettes, des comprimés, des pilules ou des dragées, à la détermination des caractéristiques physico-mécaniques de ceux-ci, telles que le poids, les dimensions, le temps de désagrégation dans un médium, ainsi que la dureté, en différentes étapes de process, en ayant recours à une unité d'alimentation (3, 6) et à une unité de transport en étoile (2) avec des chambres (5) disposées en périphérie pour loger chacune un échantillon, ainsi qu'à un testeur de dureté (13), le cas échéant à une balance (7), ainsi qu'à une unité (8) pour déterminer les dimensions de l'échantillon,
**caractérisé en ce que** l'étape de process de la mesure de la dureté d'un échantillon est désaccouplée des autres étapes de process, et que cette étape de process de la mesure de la dureté d'un échantillon est accomplie en parallèle, pendant les autres étapes de process effectuées sur un autre échantillon.

8. Procédé selon la revendication 7, **caractérisé en ce que**
pendant l'acheminement d'un échantillon dans l'étape de process de la mesure de la dureté, une pluralité d'autres échantillons à l'intérieur du dispositif de transport en étoile sont soumis aux étapes de process de la pesée et de la mesure des dimensions pendant l'accomplissement de l'étape de process de la mesure de la dureté.
